# EUROPEAN PATENT APPLICATION

(11) **EP 4 481 041 A1**
(43) Date of publication of application: **25.12.2024**
(21) Application number: 23382624.7
(22) Date of filing: 21.06.2023
(51) Int. Cl.: C12N 9/50, A61K 38/48

(54) **ATMC1 FOR CLEARING PROTEIN AGGREGATES**

(71) Applicant: Centro de Investigación en Agricenómica - CRAG, 08193 Cerdanyola Del Vallès (ES); Consejo Superior De Investigaciones Científicas - CSIC, 28006 Madrid (ES); Universitat de Barcelona, 08028 Barcelona (ES); Universitat Autònoma de Barcelona, 08193 Bellaterra (Cerdanyola del Valles) (ES)
(72) Inventor: SÁNCHEZ COLL, Nuria, 08195 SANT CUGAT DEL VALLÈS (ES); ARMENGOT MARTÍNEZ, Laia, 08241 MANRESA (ES); SALGUERO LINARES, Jose Manuel, NORWICH, NR1 3NX (GB); RUIZ SOLANÍ, Nerea, 08173 SANT CUGAT DEL VALLÈS (ES); VENTURA ZAMORA, Salvador, 08025 BARCELONA (ES); PALLARÈS GOITIZ, Irantzu, 08290 CERDANYOLA DEL VALLÈS (ES); SANTOS SUÁREZ, Jaime, 08205 SABADELL (ES)
(74) Representative: ZBM Patents - Zea, Barlocci & Markvardsen

(57) **Abstract**

The present invention provides a polypeptide having disaggregase activity for use in medicine, wherein the polypeptide comprises the sequence SEQ ID NO: 1 or SEQ ID NO: 2, or a variant thereof that retains the disaggregase activity, more particularly for use in the prevention or treatment of a disease or disorder associated with protein aggregates in a subject. The invention also provides an expression vector encoding the polypeptide for the same use, and uses and methods for eliminating or preventing the formation of a protein aggregate in a protein-containing composition. The invention also provides the use of the polypeptide for imaging a protein aggregate and for the diagnosis and prognosis of a disease associated with protein aggregates.

## Description

### Technical Field

The present invention belongs to the field of compounds capable of eliminating protein aggregates, in particular polypeptides with disaggregase activity. The polypeptides of the invention are particularly useful as a medicament for treating disorders or diseases associated with protein aggregates.

### Background Art

Proteins are the most abundant macromolecules of the cell and are critical to virtually all physiological processes. To perform their biological functions, the majority of proteins need to fold into and maintain their native conformations. Although the native conformation of a protein is determined by its amino acid sequence, the folding process is extraordinarily complex and highly prone to error, and its utility can be further limited in situations of genetic mutations, biogenetic inaccuracies, and posttranslational damages. Proteins that have adopted aberrant conformations, and the aggregates formed by them, pose a constant threat to cell viability and function. Failure to eliminate these aggregates is closely linked to the pathogenesis of various debilitating human diseases, particularly neurodegenerative diseases, including Alzheimer's disease, Parkinson's disease, and amyotrophic lateral sclerosis.

Current treatments for these diseases are mostly palliative and ineffective. No effective therapeutics exist that reverse the aberrant protein misfolding and aggregation that underlie disease. The lack of effective therapies is a cause of immense angst as these diseases are increasing in prevalence as our population ages.

Protein disaggregases -i.e., proteins capable of clearing protein aggregates- have been postulated as a potentially useful tool for treating diseases associated with protein aggregates (Shorter J., "Designer protein disaggregases to counter neurodegenerative disease", 2017, Curr Opin Genet Dev., vol. 44, pp. 1-8). However, most of disaggregases known have several drawbacks that have limited their use in the clinic or in industrial settings. For example, many are from bacterial origin, which raises concerns of harmful immune response upon their administration, or are ATP-dependent, which limits their efficiency against the extracellular aggregates associated to these diseases.

Hence, there is still a need to identify novel protein disaggregases, and in particular those useful in the treatment of diseases associated to protein aggregates.

### Summary of Invention

The present inventors have discovered a strong and specific disaggregase activity of *Arabidopsis thaliana* Metacaspase-1 (AtMC1), which makes it particularly useful as a protein disaggregase for *in vivo* and *in vitro* applications.

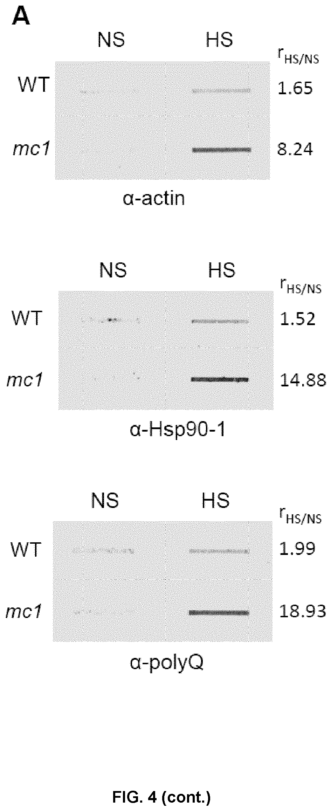

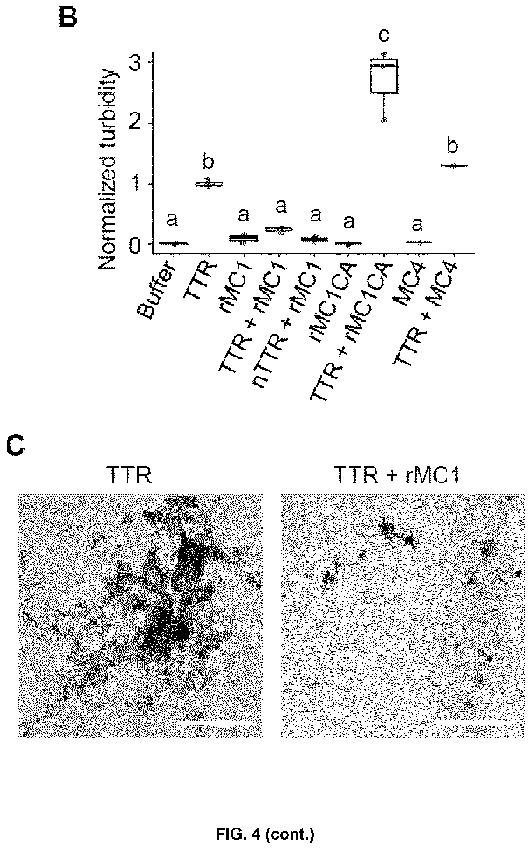

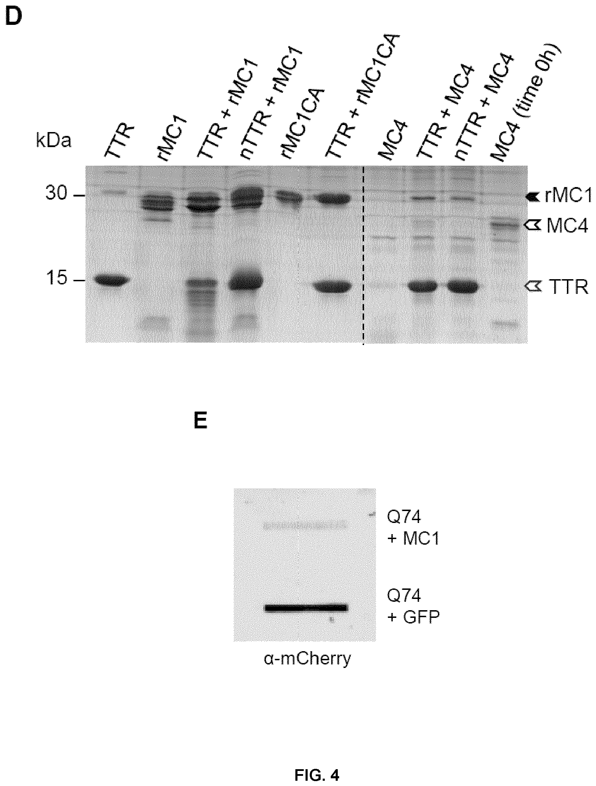

As shown in the examples below, the present inventors have discovered that AtMC1 alone can clear pathological protein aggregates, even when used extracellularly, or when the aggregates are from evolutionarily distant organisms, ranging from yeast to humans (see Figure 4). Previous to the present invention, it had been hypothesized that endogenous AtMC1 could be somehow involved in the maintenance of cellular proteostais in plants; however, it was completely unknown and unexpected that AtMC1 could have a direct effect on the clearance of aggregates, much less that it could act alone as a potent disaggregase, both inside and outside the cells, and on aggregates from very distant organisms, including humans.

Additionally, the present inventors have found that the disaggregase activity of AtMC1 is specific -it clears protein aggregates but not the functional form of proteins (see Figure 4D, lines 3 and 4). Accordingly, AtMC1 displays slower and less efficient protease activity towards typical metacaspase substrates, while showing proteolytic activity specifically directed towards aggregated protein. Notably, this specific disaggregase activity was not observed in other metacaspases from plants (see Figure 4D, lines 8 and 9).

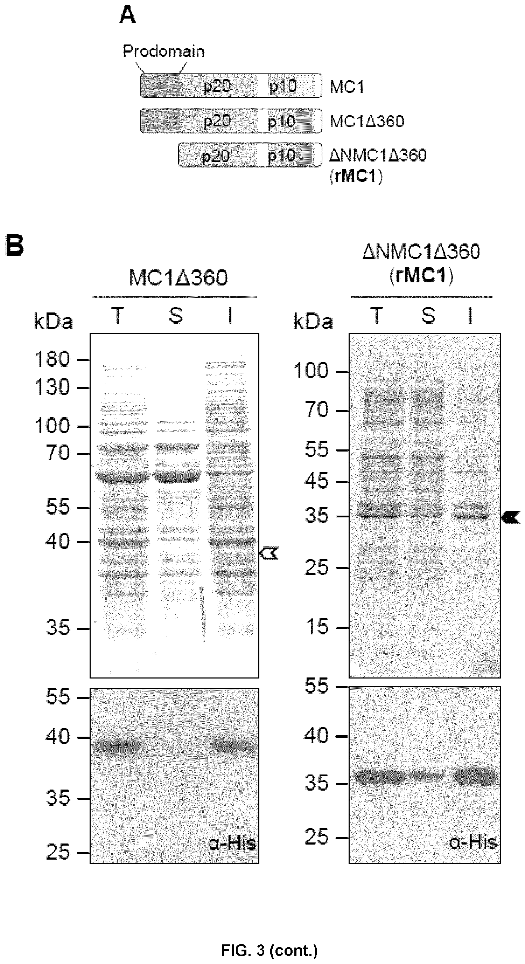

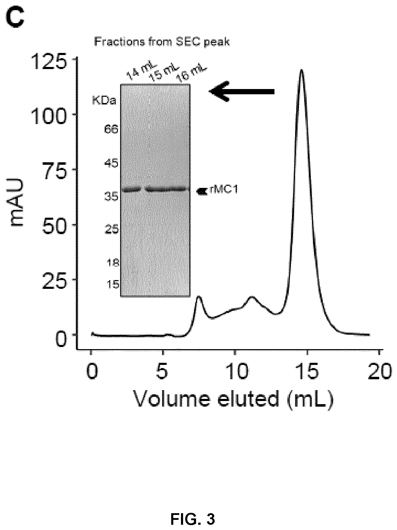

The inventors have also discovered that variants of AtMC1 lacking the N-terminal prodomain and the 360 loop maintain the disaggregase activity while improving their solubility, which facilitates their production and use (see Figure 3).

In conclusion, the present inventors have found that AtMC1 shows an exceptional combination of properties that makes it highly suitable for clearing protein aggregates, both *in vivo* and *in vitro.* Moreover, from the data provided below in several disease models (e.g. Huntington's disease, transthyretin amyloidosis) it is apparent that AtMC1 can be used as a medicament for treating human diseases associated with pathological protein aggregation.

Thus, in a first aspect, the invention provides a polypeptide having disaggregase activity for use in medicine, wherein the polypeptide comprises the sequence of SEQ ID NO: 1 (i.e., AtMC1) or SEQ ID NO: 2 (i.e., soluble AtMC1 lacking the prodomain and the 360 loop), or a variant thereof that retains the disaggregase activity.

In a second aspect, the invention provides a polypeptide having disaggregase activity for use in the prevention or treatment of a disease or disorder associated with protein aggregates in a subject, wherein the polypeptide comprises the sequence of SEQ ID NO: 1 or SEQ ID NO: 2, or a variant thereof that retains the disaggregase activity.

In a third aspect, the invention provides a nucleic acid for use in medicine, particularly in the prevention or treatment of a disease or disorder associated with protein aggregates in a subject, wherein the nucleic acid encodes a polypeptide as defined in the first and second aspects.

In a fourth aspect, the invention provides a gene construct for use in in medicine, particularly the prevention or treatment of a disease or disorder associated with protein aggregates in a subject, wherein the gene construct comprises a nucleic acid as defined in the third aspect operatively linked to an expression promoter.

In a fifth aspect, the invention provides an expression vector for use in medicine, particularly in the prevention or treatment of a disease or disorder associated with protein aggregates in a subject, wherein the expression vector comprises a gene construct as defined in the fourth aspect.

In a sixth aspect, the invention provides a host cell for use in medicine, particularly in the prevention or treatment of a disease or disorder associated with protein aggregates, in a subject, wherein the host cell comprises a nucleic acid as defined in the third aspect, a gene construct as defined in the fourth aspect, or an expression vector as defined in the fifth aspect.

In a seventh aspect, the invention provides a pharmaceutical composition comprising, together with at least one pharmaceutically acceptable excipient, diluent, or carrier, a polypeptide having disaggregase activity, wherein the polypeptide comprises the sequence of SEQ ID NO: 1 or SEQ ID NO: 2, or a variant thereof that retains the disaggregase activity; a nucleic acid that encodes the polypeptide having disaggregase activity; a gene construct comprising the nucleic acid; or an expression vector comprising the gene construct.

As shown in the examples below, the inventors have found that AtMC1 can also efficiently clear protein aggregates *in vitro,* thus it can be useful for avoiding the aggregation of proteins in non-therapeutic methods, for example in methods for the *in vitro* production of recombinant proteins.

Thus, in an eighth aspect, the invention provides a method for eliminating or preventing the formation of a protein aggregate in a protein-containing composition, the method comprising contacting the protein-containing composition with a polypeptide having disaggregase activity, wherein the polypeptide comprises the sequence of SEQ ID NO: 1 or SEQ ID NO:2, or a variant thereof that retains the disaggregase activity.

In a nineth aspect, the invention provides the use of a polypeptide having disaggregase activity for eliminating or preventing the formation of a protein aggregate in a protein-containing composition, wherein the polypeptide comprises the sequence of SEQ ID NO: 1 or SEQ ID NO: 2, or a variant thereof that retains the disaggregase activity.

In a tenth aspect, the invention provides a method for eliminating or preventing the formation of a protein aggregate in a plant, the method comprising contacting the plant with a polypeptide having disaggregase activity or a nucleic acid encoding thereof, wherein the polypeptide comprises the sequence of SEQ ID NO: 1 or SEQ ID NO:2, or a variant thereof that retains the disaggregase activity.

In an eleventh aspect, the invention provides the use of a polypeptide having disaggregase activity or a nucleic acid encoding thereof for eliminating or preventing the formation of a protein aggregate in a plant, wherein the polypeptide comprises the sequence of SEQ ID NO: 1 or SEQ ID NO: 2, or a variant thereof that retains the disaggregase activity.

As shown below, the present inventors found that AtMC1 displays specificity for protein aggregates compared to the corresponding unaggregated proteins, which makes it also useful for imaging protein aggregates both *in vivo* and *in vitro,* and also diagnosing a disease or disorder associated with protein aggregates.

Thus, in a twelfth aspect, the invention provides method for imaging a protein aggregate in a subject, the method comprising the steps of (i) administering into the subject a detectable quantity of a detectably labelled polypeptide having disaggregase activity, wherein the polypeptide comprises the sequence of SEQ ID NO: 1 or SEQ ID NO: 2, or a variant thereof that retains the disaggregase activity; and (ii) detecting the polypeptide associated with the protein aggregate.

In a thirteenth aspect, the invention provides a method for imaging a protein aggregate in a sample, the method comprising contacting the sample with a polypeptide having disaggregase activity, wherein the polypeptide is a detectably labelled polypeptide having disaggregase activity, and wherein the polypeptide comprises the sequence of SEQ ID NO: 1 or SEQ ID NO: 2, or a variant thereof that retains the disaggregase activity.

In a fourteenth aspect, the invention provides the use of a polypeptide having disaggregase activity for imaging a protein aggregate, wherein the polypeptide is a detectably labelled polypeptide comprising the sequence of SEQ ID NO: 1 or SEQ ID NO: 2, or a variant thereof that retains the disaggregase activity.

In a fifteenth aspect, the invention provides a polypeptide having disaggregase activity for use in the *in vivo* diagnosis of a disease or disorder associated with protein aggregates in a subject, wherein the polypeptide is a detectably labelled polypeptide comprising the sequence of SEQ ID NO: 1 or SEQ ID NO:2, or a variant thereof that retains the disaggregase activity.

In a sixteenth aspect, the invention provides a method for diagnosing a disease or disorder associated with protein aggregates in a sample from a subject (i.e., isolated sample from the subject), the method comprising contacting the sample with a polypeptide having disaggregase activity, wherein the polypeptide is a detectably labelled polypeptide having disaggregase activity, and wherein the polypeptide comprises the sequence of SEQ ID NO: 1 or SEQ ID NO: 2, or a variant thereof that retains the disaggregase activity.

In a seventeenth aspect, the invention provides a kit of parts for use in medicine, particularly in the prevention or treatment of a disease or disorder associated with protein aggregates, wherein the kit of parts comprises a polypeptide as defined in the first or second aspects, a nucleic acid as defined in the third aspect, a gene construct as defined in the fourth aspect, an expression vector as defined in the fifth aspect, or a host cell as defined in the sixth aspect; and instructions for its use.

In an eighteenth aspect, the invention provides a kit of parts comprising the pharmaceutical composition as defined in the seventh aspect, and instructions for its use.

### Brief Description of Drawings

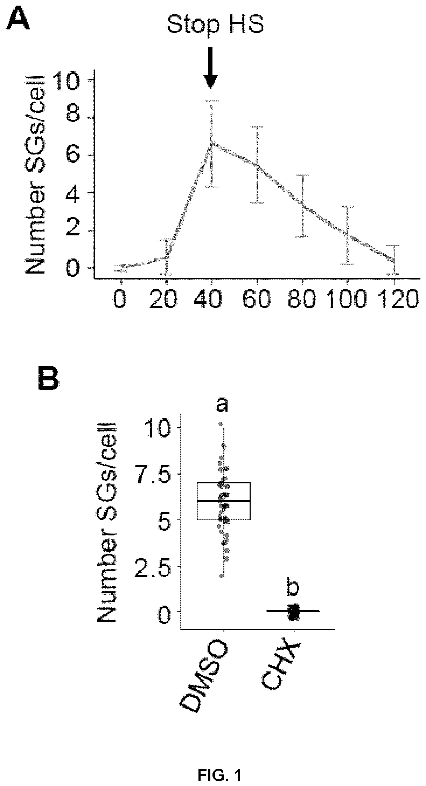

Fig. 1. MC1 is recruited to stress granules (SGs) upon heat stress. A) Kinetics of the assembly and disassembly of MC1 cytoplasmic foci. Graph shows means ± sd of three independent experiments, each including 5 seedlings. Eight to 10 cells for each seedling were analyzed for SG quantification. "Stop HS" corresponds to the time point when plants were transferred from 39 °C to 22 °C. B) Quantification of MC1-GFP foci in the experiment shown in C. Upper and lower box boundaries represent the first and third quantiles, respectively; horizontal lines mark the median and whiskers mark the highest and lowest values. Three independent experiments, each containing at least five individual measurements, were performed. Means with different letters are significantly different at P < 0.05 (one-way ANOVA).

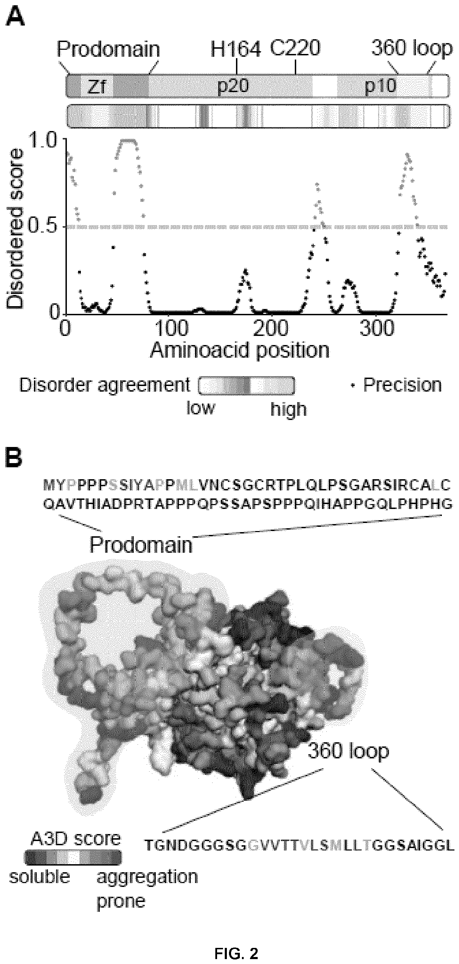

Fig. 2. MC1 contains intrinsically disordered and aggregation-prone regions. A) Prediction of intrinsically disordered regions of MC1. Top, scheme of MC1 protein structure. Zf: LSD1-Zinc finger domain within the prodomain (amino acids 1-77); H164 and C220 correspond to the amino acids of the catalytic dyad within the large p20 catalytic subunit; 360 loop (amino acids 318-346): hydrophobic loop within the small p10 catalytic subunit. Middle and Bottom, prediction of the disordered regions by D²P² (https://d2p2.pro) and DISOPRED3 (http://bioinf.cs.ucl.ac.uk/psipred), respectively. B) Aggrescan 3D structure of MC1. The amino acid sequences are of the prodomain and 360 loop are shown and the amino acids with high A3D scores (aggregation-prone) are highlighted.

Fig. 3. The prodomain and the 360 loop confer insolubility to MC1. A) Schematic representation of MC1 full length, MC1Δ360 (without 360 loop) and ΔNMC1Δ360 (without prodomain and 360 loop) domain architecture. B) SDS-PAGE Coomassie-stained gels (upper panels) and western blot analysis (lower panels) of lysates from total, soluble or insoluble fractions of E. coli cells expressing either MC1Δ360 or ΔNMC1Δ360 (rMC1). Arrow denotes expected molecular weight of each of the two MC1 variants. C) Size-exclusion chromatography of concentrated eluates obtained by nickel affinity chromatography. The inlet shows an SDS-PAGE Coomassie-stained gel of fractions 14ml to 16 mL of the eluted volume from a Superdex 75 column.

Fig. 4. MC1 can specifically clear protein aggregates *in vitro* and *in vivo* and the lack of MC1 results in protein aggregate accumulation. A) Filter trap analysis of protein extracts from five-day-old Arabidopsis mc1 or WT seedlings in control conditions (NS) or subjected to a severe heat stress (HS, 90 min at 37 °C, 90 min at 22 °C and 90 min at 45 °C). SDS resistant aggregates were detected using antibodies against actin, HSP90-1 or polyQ proteins. rHS/NS represents the ratio between HS and NS protein levels. Signal intensity of the bands was quantified using Image J. Two independent experiments were performed with similar results. B) Turbidity assays of end-point disaggregation reactions using light scattering at 360 nm. rMC1, the catalytic inactive form rMC1C220A (rMC1CA), or MC4, were co-incubated with TTR aggregates or native tetrameric TTR for 24 hours at 37°C. Recombinant MC1 proteins and TTR aggregates, incubated for the same period were also measured as controls. Data represents three individual measurements. Upper and lower box boundaries represent the first and third quantiles, respectively; horizontal lines mark the median and whiskers mark the highest and lowest values. Means with different letters are significantly different at P < 0.05 (one-way ANOVA). C) Electron microscopy images of end-point disaggregation reactions of TTR aggregates incubated with or without purified rMC1 for 24 hours at 37 °C. D) SDS-PAGE analysis of the end-point samples shown in panel C. E) Filter trap analysis showing mRFP-Q74 aggregation levels in HEK293 cells. HEK293 cells were transfected with mRFP-Q74 and GFP-MC1 or mRFP-Q74 and GFP as a control. mCherry antibody was used to detect Q74 SDS-resistant aggregates.

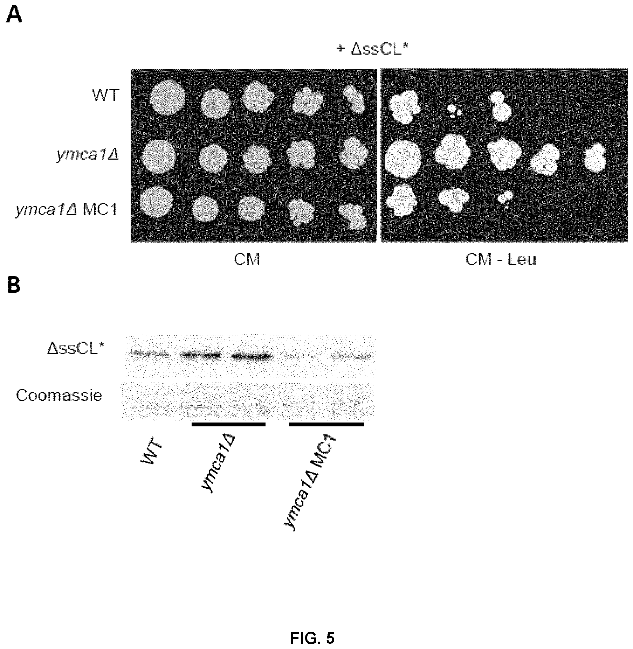

Figure 5. MC1 participates in the clearance of terminally misfolded proteins in yeast. A) Serial dilutions of wild type (WT), ymca1Δ mutant and ymca1ΔMC1-complemented cells expressing ΔssCL* were spotted on indicated media and incubated for 3 days at 30°C. Enhanced growth on plates lacking leucine (CM-Leu) indicates stabilization of ΔssCL*, whereas reduced growth indicates increased degradation. Three independent experiments were performed. B) SDS-PAGE of ΔssCL* levels of the strains shown in A.a-myc was used to detect ΔssCL*.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more". Unless indicated otherwise, definite articles used herein, such as "the" also include the plural of the noun.

As above indicated, the present invention provides a new disaggregase for use in medicine, particularly for use in the treatment or prevention of a disease or disorder associated with protein aggregates.

The term "polypeptide having disaggregase activity" refers to a polypeptide capable of eliminating and/or preventing the formation of protein aggregates, in particular insoluble protein aggregates. As used herein, the terms "peptide", "polypeptide," and "protein" are used interchangeably, and refer to a compound comprised of two or more amino acid residues covalently linked by peptide bonds.

In a particular embodiment of the first aspect, the polypeptide is for use as a medicament. In another embodiment, the polypeptide is for use in therapy or *in vivo* diagnosis.

In a particular embodiment, the polypeptide is for use in a mammal, particularly in a human.

In a second aspect, the invention provides the polypeptide for use in de treatment or prevention of a disease or disorder associated with protein aggregates.

This aspect can also be formulated as the use of the polypeptide of the invention for the manufacture of a medicament the prevention or treatment of a disease or disorder associated with protein aggregates in a subject. This aspect can also be formulated as a method for the prevention or treatment of a disease or disorder associated with protein aggregates in a subject, the method comprising administering a therapeutically effective amount of the polypeptide of the invention together with at least one pharmaceutically acceptable excipient and/or carrier to a subject in need thereof.

The term "disease or disorder associated with protein aggregates" refers to a disease or disorder characterized by the presence of detrimental aggregated proteins, typically of abnormal or misfolded proteins. Though these diseases are frequently neurodegenerative diseases, they also include diseases of other tissues, including the liver, muscle and heart, and include some cancers. Various methods for assessing whether a disease is associated protein misfolding or protein aggregates are known in the art. "Protein aggregates" refers to non-covalently linked oligomers or multimers of one or more types of proteins, which are typically characterized by an altered three-dimensional conformation of the complexed protein units with respect to the monomeric protein units and a low solubility of the complexes in aqueous solutions. A "disease" is a state of health of an animal wherein the animal cannot maintain homeostasis, and wherein if the disease is not ameliorated then the animal's health continues to deteriorate. In contrast, a "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.

The term "treatment", as used herein, unless otherwise indicated, refers to any type of therapy which is aimed at terminating, preventing, ameliorating or reducing the susceptibility to a clinical condition or existing disease as described herein, including complete curing of a disease as well as amelioration or alleviation of said disease. Thus, "treatment," "treating," and their equivalent terms refer to obtaining a desired pharmacologic or physiologic effect, covering any treatment of a pathological condition or disorder in a subject. The term "prevention" or "preventing", as used herein, means, but is not limited to, a process of prophylaxis in which a subject is exposed to the polypeptide of the invention prior to the induction or onset of the disease process.

In a particular embodiment, the protein aggregates of the disease or disorder associated with protein aggregates comprise at least one protein selected from the group consisting of prion protein, polyglutamine protein (e.g. huntingtin, androgen receptor, atrophin 1, ataxin 1, ataxin 2, ataxin 3, ataxin 7, TATA box binding protein), amyloid precursor protein (APP), alpha-synuclein, superoxide dismutase, transthyretin, tau, immunoglobulin, amyloid-A, transthyretin, a low density lipoprotein receptor, crystallin, beta 2-microglobulin, cystatin C, apolipoprotein A1, TDP-43, FUS, SOD-1, islet amyloid polypeptide, ANF, gelsolin, insulin, lysozyme, p53, and fibrinogen.

In one embodiment, the disease or disorder associated with protein aggregates is selected from the group consisting of Protein Conformational Disorders (i.e., prion disease, for example, bovine spongiform encephalopathy, kuru, Creutzfeldt-Jakob disease, variant Creutzfeldt-Jakob disease, Gerstmann-Straussler-Scheinker syndrome, fatal familial insomnia), alpha-Synucleinopathies, Polyglutamine Diseases (e.g. Huntington's disease, Spinocerebellar ataxia Type 1 (SCA1), SCA2, SCA3, SCA6, SCA7, SCA17), Serpinopathies, Tauopathies (e.g. frontotemporal dementia, Alzheimer's disease, progressive supranuclear palsy, corticobasal degeration, frontotemporal lobar degeneration) or other related diseases or disorders.

In one embodiment, the disease or disorder mediated by protein aggregates is selected from the group consisting of Alzheimer's disease, transthyretin amyloidosis, cerebral β-amyloid angiopathy, retinal ganglion cell degeneration, bovine spongiform encephalopathy, kuru, Creutzfeldt-Jakob disease, variant Creutzfeldt-Jakob disease, Gerstmann-Straussler-Scheinker syndrome, fatal familial insomnia, frontotemporal dementia, progressive supranuclear palsy, corticobasal degeration, frontotemporal lobar degeneration, frontemporal lobar degeneration, amyotrophic lateral sclerosis, Huntington's disease, familial British dementia, Familial Danish dementia, hereditary cerebral hemorrhage with amyloidosis, CADASIL, Alexander disease, Seipinopathies, familial amyloidotic neuropothy, senile systemic amyloidosis, serpinopathies, AL amyloidosis, AA amyloidosis, type II diabetes, aortic medial amyloidosis, ApoAl amyloidosis, Apoll amyloidosis, ApoAIV amyloidosis, familial amyloidosis of the Finish type, lysozyme amyloidosis, fibrinogen amyloidosis, dialysis amyloidosis, inclusion body myositis/myopathy, cataracts, cancer associated with p53 aggregates, medullary thyroid carcinoma, cardiac atrial amyloidosis, pituitary prolactinoma, hereditary lattice corneal dystrophy, cutaneous lichen amyloidosis, corneal lactoferrin amyloidosis, corneal lactoferrin amyloidosis, pulmonary alveolar proteinosis, odontogenic tumor amyloidosis, seminal vesical amyloid, cystic fibrosis, sickle cell disease, critical illness myopathy, von Hippel-Lindau disease, spinocerebellar ataxia type 1, spinocerebellar ataxia type 2, spinocerebellar ataxia type 3 (Machado-Joseph disease), spinocerebellar ataxia type 6, spinocerebellar ataxia type 7, spinocerebellar ataxia type 17, Angelman syndrome, giant axon neuropathy, and inclusion body myopathy with Paget disease of bone and frontotemporal dementia (IBMPFD). In another particular embodiment, the disease or disorder associated with protein aggregates is an amyloidosis (e.g. transthyretin amyloidosis) or a polyglutamine disease (e.g. Huntington's disease). In a more particular embodiment, the amyloidosis is transthyretin amyloidosis and/or the polyglutamine disease is Huntington's disease.

In another embodiment, the disease or disorder is a neurodegenerative disease or disorder. In another embodiment, the disease or disorder associated with protein aggregates is selected from the group consisting of Parkinson's disease, Alzheimer's disease, and Huntington's disease.

In another embodiment, the disease or disorder is associated with insoluble protein aggregates.

In one embodiment, the prevention or treatment comprises reducing the number or size of protein aggregates in the subject, and/or the rate of aggregation of the protein aggregates in the subject.

As shown in the examples below, the polypeptide of the invention is capable of clearing aggregates from very evolutionary distant organisms, including yeasts and animals.

Thus, in a particular embodiment, the subject is an animal. In a more particular embodiment, the subject is a mammal. In an even more particular embodiment, the subject is a human.

SEQ ID NO: 1 is the amino acid sequence of *Arabidopsis thaliana* Metacaspase-1, also known as AtMC1, AtMCP1b, AtMCA-la, or MC1 (NCBI accession NP_171719; version: NP_171719.2). SEQ ID NO: 2 is the amino acid sequence of a soluble version of AtMC1 with the exact same sequence except that it lacks the N-terminal prodomain (amino acids 2-77 of SEQ ID NO: 1) and the 360 loop (amino acids 318-346 of SEQ ID NO: 1). As shown in the examples below, this version maintains the disaggregase activity of the full-length protein and has increased solubility.

In a particular embodiment, the polypeptide comprises or consists of the amino acids 78-317 of SEQ ID NO:1, or a variant thereof that retains the disaggregase activity. In a more particular embodiment, the polypeptide comprises or consists of the amino acids 1, 78-317 of SEQ ID NO: 1, or a variant thereof that retains the disaggregase activity.

In a particular embodiment, the polypeptide comprises or consists of the amino acids 78-317 and 347-367 of SEQ ID NO: 1, or a variant thereof that retains the disaggregase activity. In a more particular embodiment, the polypeptide comprises or consists of the amino acids 1, 78-317, and 347-367 of SEQ ID NO:1, or a variant thereof that retains the disaggregase activity.

In an embodiment, the polypeptide comprises or consists of the amino acids 1-317 and 347-367 of SEQ ID NO: 1, or a variant thereof that retains the disaggregase activity. In another embodiment, the polypeptide comprises or consists of the amino acids 78-367 of SEQ ID NO: 1, or a variant thereof that retains the disaggregase activity. In a more particular embodiment, the polypeptide comprises or consists of the amino acids 1, and 78-367 of SEQ ID NO: 1, or a variant thereof that retains the disaggregase activity.

In a particular embodiment, the polypeptide comprises or consists of: a) the sequence of SEQ ID NO: 1, optionally lacking the amino acids 2-77 and/or 318-346; or b) a variant of SEQ ID NO: 1, optionally lacking the amino acids 2-77 and/or 318-346, that retains the disaggregase activity and/or is at least 70 %, at least 71 %, at least 72%, at least 73%, at least 74%, at least 75%, at least 76%, at least 77%, at least 78%, at least 79%, at least 80 %, at least 81 %, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identical to SEQ ID NO: 1.

In a particular embodiment, the polypeptide comprises the sequence of SEQ ID NO: 1 or SEQ ID NO: 2, or a variant thereof that retains the disaggregase activity and/or that is at least 70 %, at least 71 %, at least 72%, at least 73%, at least 74%, at least 75%, at least 76%, at least 77%, at least 78%, at least 79%, at least 80 %, at least 81 %, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identical to SEQ ID NO: 1 or SEQ ID NO: 2.

In a particular embodiment, the variant of SEQ ID NO: 1 or SEQ ID NO: 2 substantially maintains or improves the disaggregase activity of SEQ ID NO: 1 or SEQ ID NO: 2. In another embodiment, the variant of SEQ ID NO: 1 or SEQ ID NO: 2 is a functionally equivalent variant. In another embodiment, the variant of SEQ ID NO: 1 or SEQ ID NO: 2 is a catalytically active variant.

In a particular embodiment, the polypeptide consists of SEQ ID NO: 1 or SEQ ID NO: 2, or a variant thereof that retains the disaggregase activity. In a more particular embodiment, the polypeptide consists of SEQ ID NO: 1 or SEQ ID NO: 2, or a variant thereof that retains the disaggregase activity and/or that consists of a sequence at least 70 %, at least 71 %, at least 72%, at least 73%, at least 74%, at least 75%, at least 76%, at least 77%, at least 78%, at least 79%, at least 80 %, at least 81 %, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identical, to SEQ ID NO: 1 or SEQ ID NO: 2. In an even more particular embodiment, the polypeptide consists of SEQ ID NO: 1 or SEQ ID NO: 2.

Polypeptide sequence variants are well understood to those of skill in the art and can involve amino acid sequence modifications. Amino acid sequence modifications typically fall into one or more of three classes: substitutional, insertional, or deletional variants. Substitutional modifications in the sequence of polypeptide variants are typically limited or conservative, so that the sequences of the reference peptide and the variant are closely similar overall and, in many regions, identical. A variant and reference peptide can differ in amino acid sequence by one or more substitutions, additions, deletions in any combination. In some instances, a variant has no more than 75, 50, 40, 30, 25, 20, 15, 12, 10, 9, 8, 7, 6, 5, 4, 3, 2, 1 amino acid differences as compared to the reference sequence. A variant of a polypeptide can be a naturally occurring such as an allelic variant, or can be a variant that is not known to occur naturally. Non-naturally occurring variants of polypeptides may be made by mutagenesis techniques or by direct synthesis following routine methods.

In a particular embodiment, the variant of SEQ ID NO: 1 is encoded by a plant ortholog of the *Arabidopsis thaliana* MC1 gene.

In a particular embodiment, the polypeptide the polypeptide comprises the sequence of SEQ ID NO: 1, SEQ ID NO: 2, or SEQ ID NO: 5, or a variant thereof that retains the disaggregase activity.

The skilled person can readily identify variants of SEQ ID NO: 1 or SEQ ID NO: 2 that retain the disaggregase activity following routine methods without the need of inventive skill. For example, to test if a variant maintains the disaggregase activity the skilled person could carry out an *in vitro* cell-free disaggregation assay as described in the examples below. Briefly, protein aggregates (e.g. TTR aggregates) are coincubated with the variant at 37 °C in presence of DTT and CaCl₂, and sample turbidity is measured by synchronous light scattering. If sample turbidity is maintained or increased over time, the variant does not retain the disaggregase activity, whereas if sample turbidity is reduced over time, the variant retains the disaggregase activity. Disaggregation activity can also be measured by the filter trap assay, as explained in the examples below.

In the present invention the term "identical" or "identity" refers to the percentage of positions that are identical in the two sequences when the sequences are optimally aligned. If, in the optimal alignment, a position in a first sequence is occupied by the same nucleotide or amino acid as the corresponding position in the second sequence, the sequences exhibit identity with respect to that position. The percentage of identity determines the number of identical nucleotides or amino acids over a defined length in a given alignment. Thus, the level of identity between two sequences or ("percent sequence identity") is measured as a ratio of the number of identical positions shared by the sequences with respect to the number of positions compared (i.e., percent sequence identity = (number of identical positions/total number of positions compared) × 100). A gap, i.e., a position in an alignment where a nucleotide or amino acid is present in one sequence but not in the other, is regarded as a position with non-identical nucleotide or amino acid and is counted as a compared position.

A number of mathematical algorithms for rapidly obtaining the optimal alignment and calculating identity between two or more sequences are known and incorporated into a number of available software programs. For purposes of the present invention, the sequence identity between two nucleic acid or amino acid sequences is preferably determined using algorithms based on global alignment, such as the Needleman-Wunsch algorithm (Needleman and Wunsch, 1970, J. Mol. Biol. 48: 443-453), preferably implemented in the Needle program of the EMBOSS package (EMBOSS: The European Molecular Biology Open Software Suite, Rice et al., 2000, Trends Genet. 16: 276-277); or the BLAST Global Alignment tool (Altschul et al., "Basic local alignment search tool", 1990, J. Mol. Biol, v. 215, pages 403-410), using default settings. Local alignment also can be used when the sequences being compared are substantially the same length.

In a particular embodiment of the first aspect, the polypeptide is an isolated, synthetic, or recombinant polypeptide. "Isolated" means altered or removed from the natural state.

The sequences disclosed in the present invention are shown in Table 1 below:

**Table 1**

| | |
|---|---|
| SEQ ID NO: 1 (AtMC1) | MYPPPPSSIYAPPMLVNCSGCRTPLQLPSGARSIRCALCQAVTHIADPRTAPPPQPSSAPSPPPQIH APPGQLPHPHGRKRAVICGISYRFSRHELKGCINDAKCMRHLLINKFKFSPDSILMLTEEETDPYRIP TKQNMRMALYWLVQGCTAGDSLVFHYSGHGSRQRNYNGDEVDGYDETLCPLDFETQGMIVDDEI NATIVRPLPHGVKLHSIIDACHSGTVLDLPFLCRMNRAGQYVWEDHRPRSGLWKGTAGGEAISISG CDDDQTSADTSALSKITSTGAMTFCFIQAIERSAQGTTYGSLLNSMRTTIRNTGNDGGGSGGVVTT VLSMLLTGGSAIGGLRQEPQLTACQTFDVYAKPFTL |
| SEQ ID NO: 2 (ΔNMC1Δ360/ rMC1) | MGRKRAVICGISYRFSRHELKGCINDAKCMRHLLINKFKFSPDSILMLTEEETDPYRIPTKQNMRMAL YWLVQGCTAGDSLVFHYSGHGSRQRNYNGDEVDGYDETLCPLDFETQGMIVDDEINATIVRPLPH GVKLHSIIDACHSGTVLDLPFLCRMNRAGQYVWEDHRPRSGLWKGTAGGEAISISGCDDDQTSAD TSALSKITSTGAMTFCFIQAIERSAQGTTYGSLLNSMRTTIRNRQEPQLTACQTFDVYAKPFTL |
| SEQ ID NO: 3 (AtMC1 cDNA) | ATGTACCCGCCACCTCCCTCAAGCATCTACGCTCCTCCGATGCTGGTGAATTGCTCCGGTTGC CGGACGCCTCTCCAGCTCCCATCCGGCGCCCGATCTATTCGCTGCGCTCTCTGCCAGGCTGT TACTCATATCGCCGACCCTCGCACCGCCCCTCCTCCGCAACCTTCCTCCGCCCCTTCTCCGCC TCCCCAAATCCACGCGCCTCCCGGTCAGCTGCCTCACCCCCATGGCAGGAAGAGGGCCGTGA TCTGTGGCATCTCGTATCGTTTCTCTCGCCACGAGCTCAAAGGCTGCATCAACGACGCCAAGT GCATGCGTCACCTTCTCATCAACAAATTCAAATTCTCCCCAGATTCAATTCTCATGCTTACCGAG GAAGAAACTGATCCATATCGTATCCCGACCAAGCAAAACATGAGGATGGCATTGTATTGGCTC GTACAGGGATGCACAGCAGGCGACTCACTTGTCTTCCACTACTCTGGTCATGGTTCGCGTCAA AGAAACTACAACGGTGATGAAGTTGATGGCTATGATGAAACACTCTGTCCTCTGGATTTTGAAA CTCAGGGGATGATTGTAGACGATGAGATCAACGCAACCATTGTACGCCCTCTTCCACATGGTG TCAAGCTCCATTCAATTATCGATGCTTGCCATAGTGGTACCGTTCTGGATTTACCCTTCCTATGC AGAATGAACAGAGCTGGGCAGTATGTGTGGGAGGATCATCGGCCTAGGTCAGGTTTGTGGAA AGGAACTGCTGGTGGAGAAGCCATTTCAATTAGTGGATGTGATGATGATCAGACTTCGGCCGA CACATCAGCGCTGTCGAAGATCACGTCTACGGGTGCTATGACTTTCTGTTTTATTCAAGCAATT GAACGCAGCGCACAAGGCACAACCTATGGAAGCCTTCTGAATTCTATGCGCACCACAATAAGG AATACAGGGAATGATGGTGGTGGTAGTGGTGGAGTTGTGACGACTGTGCTGAGCATGCTTCTG ACAGGGGGAAGTGCGATTGGGGGATTAAGACAGGAGCCTCAACTGACTGCTTGCCAAACATTC GATGTCTATGCAAAGCCTTTCACTCTCTAG |
| SEQ ID NO: 4 (rMC1 cDNA) | ATGGGTCGCAAACGTGCGGTCATTTGTGGAATTAGCTACCGTTTTAGCCGCCATGAGCTGAAA GGATGCATCAACGATGCAAAGTGCATGCGCCATCTGCTGATCAACAAGTTTAAGTTTTCGCCG GATTCAATCCTGATGCTGACGGAGGAGGAAACAGATCCATATCGTATTCCGACTAAACAAAACA TGCGTATGGCATTGTATTGGCTGGTGCAGGGATGTACTGCCGGCGATAGCCTTGTTTTTCATTA TAGCGGTCACGGTTCGCGCCAGCGCAACTACAACGGCGATGAAGTAGACGGCTATGATGAGA CGCTTTGTCCGCTCGATTTTGAAACGCAAGGGATGATCGTAGATGATGAGATCAACGCCACGA TTGTGCGCCCGCTGCCACATGGCGTGAAACTGCATTCTATTATCGATGCGTGTCACTCTGGAA CGGTCCTGGATCTGCCGTTTCTGTGTCGCATGAACCGTGCCGGTCAGTATGTGTGGGAAGATC ACCGTCCGCGCAGTGGATTATGGAAAGGTACCGCTGGTGGGGAAGCCATCTCTATTTCCGGTT GTGATGATGACCAGACGAGCGCGGATACCTCTGCGTTGTCCAAAATTACCAGCACGGGGGCC ATGACTTTTTGTTTTATCCAGGCCATTGAACGCAGCGCCCAAGGAACTACCTACGGGAGCTTAC TGAACTCCATGCGTACTACTATTCGCAATCGTCAGGAACCGCAGCTGACCGCGTGCCAAACCT TTGATGTTTATGCCAAACCGTTTACCTTA |
| SEQ ID NO: 5 (MC1Δ360) | MYPPPPSSIYAPPMLVNCSGCRTPLQLPSGARSIRCALCQAVTHIADPRTAPPPQPSSAPSPPPQIH APPGQLPHPHGRKRAVICGISYRFSRHELKGCINDAKCMRHLLINKFKFSPDSILMLTEEETDPYRIP TKQNMRMALYWLVQGCTAGDSLVFHYSGHGSRQRNYNGDEVDGYDETLCPLDFETQGMIVDDEI NATIVRPLPHGVKLHSIIDACHSGTVLDLPFLCRMNRAGQYVWEDHRPRSGLWKGTAGGEAISISG CDDDQTSADTSALSKITSTGAMTFCFIQAIERSAQGTTYGSLLNSMRTTIRNRQEPQLTACQTFDVY AKPFTL |
| SEQ ID NO: 6 (rMC1CA) | MGRKRAVICGISYRFSRHELKGCINDAKCMRHLLINKFKFSPDSILMLTEEETDPYRIPTKQNMRMAL YWLVQGCTAGDSLVFHYSGHGSRQRNYNGDEVDGYDETLCPLDFETQGMIVDDEINATIVRPLPH GVKLHSIIDAAHSGTVLDLPFLCRMNRAGQYVWEDHRPRSGLWKGTAGGEAISISGCDDDQTSAD TSALSKITSTGAMTFCFIQAIERSAQGTTYGSLLNSMRTTIRNRQEPQLTACQTFDVYAKPFTL |
| SEQ ID NO: 7 (AtMC4) | MTKKAVLIGINYPGTKAELRGCVNDVRRMYKCLVERYGFSEENITVLIDTDESSTQPTGKNIRRALA DLVESADSGDVLVVHYSGHGTRLPAETGEDDDTGFDECIVPCDMNLITDDDFRDLVDKVPPGCRM TIISDSCHSGGLIDEAKEQIGESTKKEAEDEDESEESSSRFGFRKFLRSKVEGAIESRGFHIGGNKKD EDEAEEIETKEIELEDGETIHAKDKSLPLQTLIDILKQQTGNDNIEVGKIRPSLFDAFGDDSSPKVKKF MKVILGKLQAGNGEEGGLMGMLGKLASGFLEGKLNDEDYVKPAMQTHVGSKEEVYAGGSRGSVP LPDSGILISGCQTDQTSADATPAGKPTEAYGAMSNSIQTILEETDGEISNREMVTRARKALKKQGFT QQPGLYCHDGYANAPFIC |

The polypeptides of the invention, when used either *in vivo* or *in vitro,* can contain a cell-penetrating peptide (CPP) or a blood-brain barrier penetrating peptide (BBP) for improving their functionalities.

Thus, in a particular embodiment, the polypeptide further comprises a cell-penetrating peptide (CPP) and/or a blood-brain barrier penetrating peptide (BBP).

In the present invention the term "cell penetrating peptide" or "CPP" refers to short peptides that facilitate cellular uptake of various molecular cargos, in particular polypeptides. The term "blood-brain barrier penetrating peptide" or "BPP" refers to short peptides that facilitate cellular the transport of various molecular cargos, in particular polypeptides, through the blood-brain barrier.

The function of the CPPs are to deliver the cargo into cells, a process that commonly occurs through endocytosis with the cargo delivered to delivery vectors for use in research and medicine. CPPs typically have an amino acid composition that either contains a high relative abundance of positively charged amino acids such as lysine or arginine or has sequences that contain an alternating pattern of polar/charged amino acids and non-polar, hydrophobic amino acids. These two types of structures are referred to as polycationic or amphipathic, respectively. A third class of CPPs are the hydrophobic peptides, containing only nonpolar residues, with low net charge or have hydrophobic amino acid groups that are crucial for cellular uptake. CPP design and synthesis are well known in the art (Copolovici D. M. et al., "Cell-Penetrating Peptides: Design, Synthesis, and Applications", 2014, ACS Nano, 2014, 8 (3), pp 1972-1994).

Virtually any CPP with capacity to internalize a polypeptide in a cell, or any BPP with the capacity to transport a polypeptide thought the BBB can be used in the polypeptide of the present invention; nevertheless, in a particular embodiment, said carrier peptide is a peptide comprising a "PTD" ("protein transduction domain") segment. Illustrative non-limiting examples of proteins comprising protein transduction domains (PTDs) include the human immunodeficiency virus 1 (HIV-1) TAT ("transacting translational protein") protein, the Drosophila antennapedia homeotic transcription factor (Antp) and the herpesvirus simplex 1 (HSV-1) VP22 DNA-binding protein, although it has also been suggested that other proteins have this property of internalizing peptides in cells, such as influenza virus hemagglutinin, lactoferrin, fibroblast growth factor-1, fibroblast growth factor-2 and the Hoxa-5, Hoxb-4 and Hoxc-8 proteins.

The CPP or BPP can be located at any one of the (amino or carboxyl) terminal ends of the polypeptide of the invention.

The CPP or BPP may be linked to SEQ ID NO: 1 or 2 directly or through a spacer peptide. Virtually any peptide with structural flexibility can be used as a spacer peptide; nevertheless, illustrative non-limiting examples of said spacer peptides include peptides containing repeats of amino acid moieties, e.g., of Gly and/or Ser, or any other suitable repeat of amino acid moieties.

In one embodiment, the polypeptide further comprises a targeting domain, which targets the polypeptide to a desired location. For example, the targeting domain is directed to bind to a protein or protein aggregate associated with a disease or disorder. In some embodiments, the targeting domain comprises a peptide, nucleic acid, small molecule, or the like, which has the ability to bind to the targeted cell, protein, or protein aggregate. For example, in one embodiment, the targeting domain comprises an antibody or antibody fragment which binds to a targeted cell, protein, or protein aggregate.

In another embodiment, the polypeptide further comprises a secretory signal peptide to direct secretion of the polypeptide to the extracellular environment. The secretory signal peptide targets the polypeptide for translocation across the endoplasmic reticulum membrane and into the secretory pathway.

The polypeptide comprising SEQ ID NO:1 or SEQ ID NO: 2, or a variant thereof, may be administered directly as a polypeptide, or it can be expressed inside target cells of interest by means of gene therapy. To this aim the invention also provides, in a third aspect, a nucleic acid that encodes the polypeptide as defined in the first and second aspects, wherein the nucleic acid is for use in medicine, particularly in the prevention or treatment of a disease or disorder associated with protein aggregates in a subject.

The term "nucleic acid that encodes a polypeptide" is to be understood, in particular, as the mRNA coding for said polypeptide or the cDNA sequence resulting from the reverse transcription (RT-PCR) of the mRNA coding for said polypeptide.

This aspect can also be formulated as the use of the nucleic acid as defined above for the manufacture of a medicament for the prevention or treatment of a disease or disorder associated with protein aggregates in a subject. The present invention also relates to a method for prevention or treatment of a disease or disorder associated with protein aggregates in a subject, comprising administering a therapeutically effective amount of the nucleic acid as defined above, together with pharmaceutically acceptable excipients or carriers, in a subject in need thereof, including a human.

In a particular embodiment of the second aspect, the nucleic acid sequence comprises, consists essentially of, or consists of, SEQ ID NO: 3 or SEQ ID NO: 4.

In one embodiment, the nucleic acid sequence comprises of the sequence of SEQ ID NO: 3 or SEQ ID NO: 4, or a variant thereof at least 70 %, at least 71 %, at least 72%, at least 73%, at least 74%, at least 75%, at least 76%, at least 77%, at least 78%, at least 79%, at least 80 %, at least 81 %, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identical to SEQ ID NO: 3 or SEQ ID NO: 4, optionally that retains the disaggregase activity. The skilled person would appreciate that a nucleic acid variant of SEQ ID NO: 3 or SEQ ID NO: 4 that retains the disaggregase activity refers to a nucleic acid that encodes a protein that retains the disaggregase activity.

In a more particular embodiment, the nucleic acid sequence consists of the sequence of SEQ ID NO: 3 or SEQ ID NO: 4, or a variant thereof consisting of a sequence at least 70 %, at least 71 %, at least 72%, at least 73%, at least 74%, at least 75%, at least 76%, at least 77%, at least 78%, at least 79%, at least 80 %, at least 81 %, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identical to SEQ ID NO: 3 or SEQ ID NO: 4, optionally that retains the disaggregase activity. The skilled person would appreciate that a nucleic acid variant of SEQ ID NO: 3 or SEQ ID NO: 4 that retains the disaggregase activity refers to a nucleic acid that encodes a protein that retains the disaggregase activity.

In a fourth aspect, the invention provides a gene construct for use in medicine, particularly in the prevention or treatment of a disease or disorder associated with protein aggregates in a subject, wherein the gene construct comprises a nucleic acid sequence as defined in the third aspect operatively linked to an expression promoter.

This aspect can also be formulated as the use of a gene construct as defined above for the manufacture of a medicament for the prevention or treatment of a disease or disorder associated with protein aggregates in a subject. The present invention also relates to a method for the prevention or treatment of a disease or disorder associated with protein aggregates in a subject. comprising administering a therapeutically effective amount of a gene construct as defined above, together with pharmaceutically acceptable excipients or carriers, in a subject in need thereof, including a human.

In a particular embodiment of the fourth aspect, the expression promoter operatively linked is selected from the group consisting of a constitutive expression promoter, an inducible promoter, and a tissue- or cell-specific expression promoter (e.g. neuron-specific expression promoter). In a more particular embodiment, the gene construct according to the invention comprises a promoter, the sequence of SEQ ID NO: 3 or 4, and a poly(A) region.

All these gene constructs are able to express the protein of interest once inside the cell.

In order to facilitate administration of the constructs, the invention also provides, in a fifth aspect, an expression vector for use in medicine, particularly in the prevention or treatment of a disease or disorder associated with protein aggregates in a subject, wherein the expression vector comprises the gene construct as defined in the third aspect, and thus comprising the nucleic acid sequence of the second aspect coding for the polypeptide of the first aspect operatively linked to an expression promoter.

This aspect can also be formulated as the use of the expression vector as defined above for the manufacture of a medicament for the prevention or treatment of a disease or disorder associated with protein aggregates in a subject. The present invention also relates to a method for the prevention or treatment of a disease or disorder associated with protein aggregates in a subject, comprising administering a therapeutically effective amount of the expression vector as defined above, together with pharmaceutically acceptable excipients or carriers, in a subject in need thereof, including a human.

In a particular embodiment of the fifth aspect, the expression vector is a viral vector.

In a particular embodiment of the fifth aspect, the viral vector is selected from the group consisting of adenoviral vector, adeno-associated viral (AAV) vector, baculovirus vector, herpes simplex virus (HSV) vector, retroviral vector, lentiviral vector, vaccinia viral vector, and RNA virus vector. In a more particular embodiment, the viral vector is an adeno-associated virus. In a particular embodiment, it is an adeno-associated virus of serotype selected from the group consisting of AAV1, AAV2, AAV4, AAV5, AAV6, AAV7, AAV8, AAV9, AAVrh10, PHPeB, and 9P31. Additional delivery and expression systems are known in the art and are intended to be encompassed herein.

The skilled person knows how to produce the polypeptide, nucleic acid, gene construct, and expression vector for use according to the invention by routine methods well known in the art, for example, by chemical synthesis, by recombinant expression techniques, or by cleavage from a longer polypeptide or nucleic acid, without exercising any inventive skill. The sequence of a peptide or a nucleic acid may be confirmed by sequencing.

The polypeptides of the invention can be post-translationally modified. For example, post-translational modifications that fall within the scope of the present invention include signal peptide cleavage, glycosylation, acetylation, isoprenylation, proteolysis, myristoylation, protein folding and proteolytic processing, etc.

In one embodiment of any of the aspects above, the polypeptide, the nucleic acid, the gene construct, or the expression vector is administered in the form of a composition.

In another embodiment of any of the aspects above, the polypeptide, the nucleic acid, the gene construct, or the expression vector is administered in the form of a pharmaceutical composition together with at least one pharmaceutically acceptable excipient, diluent or carrier.

As above indicated, in a seventh aspect, the invention provides a pharmaceutical composition comprising the polypeptide, the nucleic acid, the gene construct, or the expression vector.

The expression "pharmaceutical composition" encompasses both compositions intended for human as well as for non-human animals. The skilled in the art understands that a pharmaceutical composition must comprise a therapeutically effective amount of the active compound. The expression "therapeutically effective amount" as used herein, refers to the amount of polypeptide, nucleic acid, gene construct, or expression vector that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The expression "pharmaceutically acceptable excipient, diluent or carrier" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and non-human animals without excessive toxicity, irritation, allergic response, immunogenicity or other problems or complications commensurate with a reasonable benefit/risk ratio.

Examples of suitable pharmaceutically acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The relative amounts of the active ingredient, the pharmaceutically acceptable excipient, and/or any additional ingredients in a pharmaceutical composition of the invention will vary, depending upon the identity, size, and/or condition of the subject treated and further depending upon the route by which the composition is to be administered.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Excipients such as coloring agents, coating agents, sweetening, and flavoring agents can be present in the composition, according to the judgment of the formulator.

The pharmaceutical compositions containing the polypeptide, nucleic acid, gene construct, or expression vector can be presented in any dosage form, for example, solid or liquid, and can be administered by any suitable route, for example, oral, parenteral, rectal, topical, intranasal, intraocular, intraperitoneal, sublingual, intraventricular route, for which they will include the pharmaceutically acceptable excipients necessary for the formulation of the desired dosage form, for example, topical formulations (ointment, creams, lipogel, hydrogel, etc.), eye drops, aerosol sprays, injectable hydrogels, injectable solutions, osmotic pumps, etc.

Exemplary diluents include, but are not limited to, calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, corn-starch, powdered sugar, and combinations thereof.

Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linked polyvinylpyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (Veegum), sodium lauryl sulfate, quaternary ammonium compounds, and combinations thereof.

Exemplary binding agents include, but are not limited to, starch (e.g., corn-starch and starch paste); gelatin; sugars (e.g., sucrose, glucose, dextrose, dextrin, molasses, lactose, lactitol, mannitol); natural and synthetic gums (e.g., acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, polyvinylpyrrolidone), magnesium aluminium silicate (Veegum), and larch arabogalactan); alginates; polyethylene oxide; polyethylene glycol; inorganic calcium salts; silicic acid; polymethacrylates; waxes; water; alcohol; and combinations thereof.

Exemplary preservatives may include antioxidants, chelating agents, antimicrobial preservatives, antifungal preservatives, alcohol preservatives, acidic preservatives, and other preservatives. Exemplary antioxidants include, but are not limited to, alpha tocopherol, ascorbic acid, ascorbyl palmitate, ascorbyl stearate, ascorbyl oleate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and combinations thereof.

Exemplary lubricating agents include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, and combinations thereof.

In a particular embodiment, the composition or the pharmaceutical composition further comprises a nanoparticle encapsulating the polypeptide, the nucleic acid, the gene construct, or the expression vector. In a more particular embodiment, the nanoparticle is a lipid nanoparticle. The skilled person would appreciate that the nanoparticle is to be biocompatible and protect the active ingredient from degradation.

The term "nanoparticle" as used herein, refers to a particle with at least two dimensions at the nanoscale, particularly with all three dimensions at the nanoscale, where the nanoscale is the range about 1 nm to about 300 nm. Particularly, when the nanoparticle is substantially rod-shaped with a substantially circular cross-section, such as a nanowire or a nanotube, the "nanoparticle" refers to a particle with at least two dimensions at the nanoscale, these two dimensions being the cross-section of the nanoparticle.

Biodegradable nanoparticle delivery systems that increase intracellular uptake, e.g., polymeric and surface modified nanoparticles can also be used. Examples include poly DL-lactide-co-glycolide (PLGA) nanoparticles, e.g., surface-modified with known surface-modifying agents, such as heparin, dodecylmethylammonium bromide (DMAB), DEAE-Dextran, lipofectin, and fibrinogen, among others.

The term "lipidic nanoparticle" as used herein, refers to a nanoparticle whose membrane is totally made of lipids. Suitable lipids include, without limitation, phospholipids such as phosphatidylcholines ("PC's"), phosphatidylethanolamines ("PE's"), phosphatidyilserines ("PS's"), phosphatidylglycerols ("PG's"), phosphatidylinositols ("PI's") and phosphatidic acids ("PA's"). Such phospholipids generally have two acyl chains, these being either both saturated, both unsaturated or one saturated and one unsaturated; said chains include, without limitation: myristate, palmitate, stearate, oleate, linoleate, linolenate, arachidate, arachidonate, behenate and lignocerate chains. Phospholipids can also be derivatized, by the attachment thereto of a suitable reactive group. Such a group is generally an amino group, and hence, derivatized phospholipids are typically phosphatidylethanolamines. The different moieties suited to attachment to PE's include, without limitation: acyl chains, useful for enhancing the fusability of liposomes to biological membranes; peptides, useful for destabilizing liposomes in the vicinity of target cells; biotin and maleimido moieties, useful for linking targeting moieties such as antibodies to liposomes; and various molecules such as gangliosides, polyalkylethers, polyethylene glycols and organic dicarboxylic acids. Other lipids which can constitute the membrane of the nanoparticle include, but are not limited to, cholesterol and 1,2-Dioleoyl-sn-Glycero-3-Phosphocholine (DOPC).

In one embodiment, the lipidic nanoparticle is selected from the group consisting of liposomes and solid-lipid nanoparticle. In another embodiment, the lipidic nanoparticle is a liposome.

The term "solid lipid nanoparticle" refers to particles, typically spherical, with an average diameter from 10 to 1000 nanometers. Solid lipid nanoparticles possess a solid lipid core matrix that can solubilize lipophilic molecules. The lipid core is stabilized by surfactants (emulsifiers). The term lipid is used here in a broader sense and includes triglycerides (e.g. tristearin), diglycerides (e.g. glycerol bahenate), monoglycerides (e.g. glycerol monostearate), fatty acids (e.g. stearic acid), steroids (e.g. cholesterol), and waxes (e.g. cetyl palmitate). All classes of emulsifiers (with respect to charge and molecular weight) have been used to stabilize the lipid dispersion.

In the present invention, the term "liposome" is to be understood as a self-assembling structure comprising one or more lipid bilayers, each of which comprises two monolayers containing amphipathic lipid molecules oppositely oriented. Amphipathic lipids comprise a polar (hydrophilic) headgroup region covalently linked to one or two non-polar (hydrophobic) acyl chains. Energetically unfavorable contacts between the hydrophobic acyl chains and the surrounding aqueous medium induce the amphipathic lipid molecules to arrange themselves such that their polar headgroups are oriented towards the bilayer's surface, while the acyl chains reorient towards the interior of the bilayer. An energetically stable structure is thus formed in which the acyl chains are effectively shielded from coming into contact with the aqueous environment. Liposomes can have a single lipid bilayer (unilamellar liposomes,"ULVs"), or multiple lipid bilayers (multilamellar liposomes,"MLVs"or"SPLVs"). Each bilayer surrounds, or encapsulates, an aqueous compartment. Given this encapsulation of aqueous volume within a protective barrier of lipid molecules, liposomes are able to sequester encapsulated molecules, e. g., nucleic acids, away from the degrading effects of factors, e. g., nuclease enzymes, present in the external environment. Liposomes can have a variety of sizes, e. g., an average diameter as low as 25 nm or as high as 10,000 nm or more. Size is affected by a number of factors, e. g., lipid composition and method of preparation, well within the purview of ordinarily skilled artisans to determine and account for, and is determined by a number of techniques, such as quasi-elastic light scattering, also within the skilled person in the art knowledge. Various methodologies, also well-known to those skilled in the art, such as sonication, or homogenization, and milling, can be used to prepare liposomes of a smaller size from larger liposomes. Extrusion can be used to size reduce liposomes, that is to produce liposomes having a predetermined mean size by forcing the liposomes, under pressure, through filter pores of a defined, selected size. Tangential flow filtration can also be used to regularize the size of liposomes, that is, to produce a population of liposomes having less size heterogeneity, and a more homogeneous, defined size distribution.

The polypeptide, nucleic acid, gene construct, or expression vector for use according to the invention can be encapsulated within the particle using well-known methods in the state of the art.

In one embodiment, the nanoparticle also protects the polypeptide, nucleic acid, gene construct, or expression vector for use according to the invention against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using standard techniques, or obtained commercially.

In another embodiment, the polypeptide, the nucleic acid, the gene construct, or the expression vector, is administered in combination with another active agent. Suitable active agents to be administered in combination with a compound of the invention are, for example, drugs for treating neurodegenerative diseases.

As above indicated, the invention provides in the eight, nineth, tenth, and eleventh aspects further uses of AtMC1 as disaggregase. All the embodiments above disclosed, particularly those related to the sequence of the polypeptide, and the aggregates, are also meant to apply to these aspects.

In one embodiment of the eighth, nineth, tenth, and eleventh aspects, the protein aggregate is a pathogenic protein aggregate or a non-pathogenic protein aggregate. In another embodiment, the protein aggregate is an insoluble protein aggregate. In a more particular embodiment, the protein aggregate is a pathogenic insoluble protein aggregate.

In In one embodiment of the eighth aspect, the method is an *in vivo* or ex *vivo* method. In another embodiment, the protein-containing composition is a biological or a non-biological sample. In another embodiment, the sample is an isolated sample from a subject. In another embodiment, the sample is a cell culture. As used herein, a "protein-containing composition" refers to any composition comprising at least one protein. The protein-containing composition can contain one type of proteins or various types of proteins.

In In one embodiment of the eighth and nineth aspects, the method is a cell-free method. In another embodiment, the protein-containing composition is a sample, particularly is a cell-free sample.

As above explained, the invention provides in the aspects tenth to sixteenth, methods for imaging (i.e., detecting the presence of) protein aggregates, and diagnosing a disease or disorder associated with protein aggregates, that involve the use of detectably labelled polypeptide comprising SEQ ID NO: 1 or SEQ ID NO: 2, or variants thereof. All the embodiments above disclosed, particularly those related to the sequence of the polypeptide, the aggregates, and the diseases, are also meant to apply to these aspects.

As used herein, a "detectably labelled polypeptide" refers to a polypeptide the comprises one or more detectable labels. The skilled person would appreciate that various labels known in the art can be attached to the polypeptide using standard techniques for labeling proteins. Examples of labels include fluorescent labels and radiolabels. There are a wide variety of radiolabels that can be used, but in general the label is often selected from radiolabels including, but not limited to, ^{1 S}F, ¹¹C, and ¹²³I. These and other radioisotopes can be attached to the protein using well known chemistry. In one embodiment, the label is detected using positron emission tomography (PET). However, any other suitable technique for detection of labels, in particular radioisotopes, may be used.

In one embodiment of the twelfth aspect, the subject is a mammal, particularly a human.

In one embodiment of the thirteenth aspect, the sample is an *in vitro* or ex *vivo* sample. In another embodiment, the sample is a cell-free sample.

In one embodiment of the fifteenth aspect, the *in vivo* diagnosis comprises (i) administering into the subject a detectable quantity of the detectably labelled polypeptide having disaggregase activity, and (ii) detecting the polypeptide associated with the protein aggregate, wherein the subject is diagnosed with a disease or disorder associated with protein aggregates when the detectably labelled polypeptide is bound to a protein aggregate.

In one embodiment of the sixteenth aspect, the method further comprises detecting the polypeptide associated with a protein aggregate in the sample, wherein the subject is diagnosed with a disease or disorder associated with protein aggregates when the detectably labelled polypeptide is bound to a protein aggregate.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### Examples

### 1. Materials and methods

### Materials used and growth conditions

All experiments were performed using *Arabidopsis thaliana* Columbia-0 (Col-0) ecotype.. The single mutant *mc1* has been previously described (GK-096A10; Coll *et al.,* 2010). All seeds were surface-sterilized with 35% NaClO for 5 min and washed five times for 5 min with sterile dH₂O. Sterile seeds were sown in solid ½ Murashige and Skoog (MS) medium with vitamins and stratified 48 h at 4 °C. Plants were grown vertically under long day (LD) conditions (16-h light/8-h dark) at 22 °C. For dark-induced senescence studies, one-week-old seedlings were transferred into soil and grown for an additional 2 weeks under LD conditions.

### Plasmid construction

To generate *Pro35S::MC1-GFP* construct, the coding sequence and native promoter (approximately 1kb) of Arabidopsis MC1 (*AT1G02170*) were amplified from Col-0 cDNA and genomic DNA, respectively. Plasmids were assembled through GreenGate cloning .

For purification of recombinant MC1 (rMC1), the MC1 coding sequence lacking the 360 loop and with or without the prodomain (MC1Δ360 [SEQ ID NO: 5] or ΔNMC1Δ360/rMC1 [SEQ ID NO: 2]) (Fig. 2) were synthesized (Twist Bioscience) with codon optimization for expression in *Escherichia coli.* Synthetic sequences contained Ndel and Xhol restriction sites at the 5'and 3'ends of the sequence, respectively. Both synthetic genes and destination vector pET28 b(+) were cut with Ndel and Xhol and subsequently ligated so that an N-terminal 6xHis tag precedes the start site of the MC1 variants. QuickChange Site-Directed Mutagenesis (Agilent Technologies) was used to cause point mutations in the catalytic site of rMC1 to generate rMC1CA (SEQ ID NO: 6).

To complement the yeast metacaspase mutant strain *ymca1Δ* with Arabidopsis MC1 (AT1G02170), it was constructed a gene replacement cassette by PCR-directed homologous recombination as previously described (Gardner and Jaspersen, 2014). The gene replacement cassette with *Arabidopsis* MC1 was transformed into *the ymca1Δ* mutant strain KanMX4.

### HEK293T cells transfection and protein extraction

The MC1 (*AT1G02170*) gene was codon-optimized for expression in animal cells and synthetized (SEQ ID NO: 3) (Twist Bioscience). To generate *ProCMV::GFP-MC1,* the synthetic gene was cloned in the pDEST-CMV-N-GFP vector by Gateway technology (Addgene). *ProCMV::mRFP-Q74* (Balaji et al., 2022), *ProCMV::GFP-MC1* and *ProCMV::GFP* (Llamas et al., 2022) were used for transfection of HEK cells (CRL-1573) following the protocol previous described (Llamas et al., 2022). After 72 h of incubation, cells were lysed in non-denaturing native lysis (300 mM NaCl, 100 mM Hepes pH 7.4, 2 mM EDTA, 2% Triton X-100) supplemented with 1X plant protease inhibitor (Merck), scraped from the tissue culture plates, and homogenized through a syringe needle (27G). Samples were centrifuged at 10,000 × g for 10 min at 4 °C and supernatant was collected. Protein concentration was determined with the Pierce BCA Protein Assay Kit (Thermo Fisher).

### Yeast strains and spot dilution assays

Yeast media preparation and molecular biology techniques were carried out using standard methods (Lázaro-Silva et al., 2015). All experiments were done using the genetic background of *Saccharomyces cerevisiae* strain BY4741. To test the capacity of each yeast strain to remove misfolded proteins, yeast cells were transformed with the plasmid pFE15 encoding the fusion construct ΔssCL∗myc (Eisele and Wolf, 2008). Growth phenotypes were assessed with spot dilution assays. Ten-fold serial dilutions were made, ranging from undiluted to a 10⁴ dilution. Five µl of each dilution were spotted onto the corresponding selective media (-ura or -ura -leu plates) and plates were incubated for at least 3 days before images were taken

### Protein purification

*E. coli* OverExpress C41 (DE3) Chemically Competent Cells from BioCat GmbH (Heidelberg, Germany) or *E.coli* BL21 strain containing the pBB542 vector (de Marco *et al.,* 2007) were transformed with expression plasmids and grown in either autoinduction media or LB, respectively. Cells were grown first at 37 °C with continuous shaking until OD₆₀₀ reached 0.6 and then transferred to 25 °C for overnight growth. In the case of expression in *E*. c*oli* Chaperone Competent Cells BL21, Isopropyl β-D-1-thiogalactopyranoside (IPTG) at 1 mM concentration was added to 400 ml cell cultures when transferred to 25 °C to induce protein expression. The pellet from overnight cultures was resuspended in 20 mM HEPES, pH 7.5, 500 mM NaCl and sonicated on ice. A centrifugation of lysates at 25,000 × g for 20 min was performed to remove cell debris and insoluble proteins. Soluble lysate was filtered through 0.45 µM sterile filters and loaded into a 5 mL nickel ion HisTrap purification column (Cytiva, Marlborough, MA, USA). Washes of the columns were performed with 20 mM HEPES, pH 7.5, 500 mM NaCl and 20 mM imidazole. Elution of proteins was performed by increasing imidazole concentrations up to 250 mM. The cleanest elutions were concentrated using Amicon filters and loaded onto a Superdex 75 size-exclusion chromatography column (GE Healthcare Life Sciences, Chicago, IL, USA) connected to an AKTA FPLC system. The Superdex 75 column was equilibrated in 20 mM HEPES, pH 7.5, 500 mM NaCl. A flow rate of 0.75 ml per minute was used to separate proteins. Samples belonging to the most prominent peaks were kept and loaded onto an SDS-PAGE gel to verify the purity of the samples. MC4 (SEQ ID NO: 7) is described in Vercammen *et al.,* 2004.

### Enzymatic activity assays

Protease activity was measured by quantification of the fluorescence intensity released from the AMC (7-amino-4-methylcoumarin) group of the fluorogenic substrate Z-FR-AMC (PeptaNova, Sandhausen, Germany) at 383 nm and 455 nm excitation and emission wavelengths, respectively, in a Tecan Infinite M200 Microplate Reader System (Männedorf, Switzerland). All proteolytic assays were performed in 20 mM HEPES (pH 7.0) containing 150 mM NaCl, varying CaCl₂ concentrations and 5 mM DTT. For estimation of pH optima, buffers containing 100 mM acetate (pH 4-pH 5.5), 100 mM MES (pH 6-pH 6.5), 100 mM HEPES (pH 7.0-pH 8.0), 100 mM Tris (pH 8.5-pH 9) and 100 mM CAPS (pH 9.5-pH 11) were used. 0.2 µg of recombinant protease was used and the concentration of fluorogenic substrates was 5 µM.

### Preparation of TTR aggregates

TTR was expressed and purified following previously described procedures (Pinheiro *et al.,* 2021). Briefly, TTR aggregation was induced by mixing 7 µM of purified TTR with an equal volume of 400 mM sodium acetate, 200 mM KCI, pH 4.4, obtaining a final TTR concentration of 3.5 µM. Samples were incubated for 72 h at 37 °C in quiescent conditions. Aggregated samples were centrifuged at 20,000 × g for 1 h to recover the insoluble material that was subsequently resuspended in 20 mM HEPES 150 mM, NaCl pH 7.5 to a concentration of 100 µM.

### In vitro cell-free disaggregation assay

End-point disaggregation reactions were performed by coincubating TTR aggregates at a concentration of 7 µM with 0.25 mg mL-1 of protease at 37 °C in presence of 5 mM DTT and 5 mM CaCl₂. Protease disaggregation was monitored using sample turbidity, SDS-PAGE and transmission electron microscopy.

### Turbidity assay

Sample turbidity was monitored as an indicator of the amount of aggregated material using synchronous light scattering. The spectra were recorded in a JASCO Spectrofluorometer FP-8200 with an excitation wavelength of 360 nm, and emission range from 340 to 380 nm. Excitation and emission bandwidth were set to 5 nm. The light scattered at 360 nm was used as a measure of turbidity.

### Protein extraction and immunoblotting

Five hundred milligrams of leaf material were mixed with 2 ml of extraction buffer (50 Mm HEPES pH 7.3, 150 mM NaCl, 0.5% Nonidet P-40, 10% glycerol, 1 mM EDTA pH 8, 5 mM DTT, 1% PVPP and 1× Protease inhibitor cocktail (Sigma, P599)) and centrifuged for 10 min at 14,000 × g at 4 °C. 5× Laemmli sample buffer was added to 100 µl supernatant and boiled for 5 min. Equal amounts of supernatant were loaded on 12% SDS-PAGE gels. Antibodies used for immunoblotting were as follows: α-GFP-HRP (1:5,000 Milteny Biotec), α-RFP-HRP (1:5,000 Abcam), α-myc (1:10,000, Sigma-Aldrich, α-actin (dilution 1:5,000, Agrisera), α-Hsp90-1 (1:2,000 Abcam) and α-polyQ (1:1,000 Merck).

### Filter trap assay

Protein extracts were obtained with native lysis buffer (300 mM NaCl, 100 mM HEPES pH 7.4, 2 mM EDTA, 2% Triton X-100) supplemented with EDTA-free protease inhibitor cocktail. When processing plant protein extracts 1X plant protease inhibitor (Merck) was added to native lysis buffer. In experiments with HEK cells, cells were homogenized by passing 7 times through syringe needle (27 G). Cellular debris was removed by several centrifugation steps at 8,000 × g for 10 min at 4 °C. Supernatant was recollected and protein concentration determined with the Pierce BCA Protein Assay Kit (Thermo Fisher). A cellulose acetate membrane filter (GE Healthcare Life Sciences) was placed in a slot blot apparatus (Bio-Rad) coupled to a vacuum system. The membrane was equilibrated with 3 washes with equilibration buffer (native buffer supplemented with 0.5% SDS). Approximately 150 µg of protein extract was supplemented with SDS at a final concentration of 0.5% and loaded and filtered through the membrane. Then, the membrane was washed three times with 0.2% SDS. The membrane was blocked in 3% BSA in TBST for 30 min followed by 3 washes with TBST. The membrane was incubated with indicated antibody and then washed 3 times for 5 min and incubate with secondary antibodies in TBST 3% BSA for 30 min. The membrane was developed using an Odissey DLx (Licor). Extracts were also analyzed by SDS-PAGE and western blotting to determine loading controls.

### Heat treatments

Thermotolerance assays were performed using 5-day-old seedlings. Seedlings were grown at 22 °C for 5 days, put in a hot-air incubator set at 37 °C for 90 min, put in a growth chamber set at 37 °C for 90 min, incubated at 45 °C for 90 min and allowed to recover at 22 °C for 8 days. The percentages of seedlings in different phenotypic classes were calculated based on results from three biological replicates. In each biological replicate, at least 50 seedlings were used for each genotype.

### Bioinformatic analyses

Intrinsically disordered regions of MC1 were predicted using the D2P2 database (D²P², http://d2p2.pro/, Oates *et al.,* 2013) and DISOPRED3 (http://bioinf.cs.ucl.uk/psipred, Jones and Cozzetto, 2015). LLPS predisposition was evaluated using the PSPredictor tool (http://www.pkumdl.cn:8000/PSPredictor/, Chu *et al.,* 2022) Analysis of the aggregation propensity of MC1 amino acids was performed with Aggrescan3D (http://biocomp.chem.uw.edu.pl/A3D/; Zambrano *et al.,* 2015) using as input file the Alphafold2 (https://alphafold.ebi.ac.uk/, Jumper *et al.,* 2021) predicted MC1 structure.

### Statistical analysis

All quantification analyses and statistical tests were performed with R software. T-test was used to compare the significance of differences between two experimental groups. For comparing the significance of differences between multiple experimental groups, one-way ANOVA or Klustal-Wallis one-way analysis were performed as indicated in each experiment. Different letters show statistically significant differences between samples.

### 2. Results

### MC1 dynamically localizes to cytoplasmic stress granules upon acute proteotoxic stress

To gain a deeper understanding into MC1 (AtMC1) function, transgenic lines of *Arabidopsis thaliana* expressing MC1 tagged with a green fluorescent protein under the control of the 35S promoter in the *mc1* mutant background were generated (*mc1 Pro35S::MC1-GFP*) and evaluated its subcellular localization. Under basal conditions MC1 showed a diffuse pattern in both cytoplasm and nucleus. Heat stress treatment (39°C for 40 min) resulted in rapid formation of dynamic cytoplasmic puncta that grew in size over time and disappeared shortly after returning the plants to non-stress conditions (Fig. 1A). The same heat-responsive relocalization pattern was observed when *MC1-GFP* was expressed under the control of its native promoter. To evaluate if such puncta correspond to stress granules we used cycloheximide, an inhibitor of translational elongation that prevents SG assembly and forces the disassembly of existing SGs. Application of cycloheximide blocked the appearance of the observed heat stress-induced puncta (Fig. 1B).
Together, these data demonstrate that MC1 dynamically re-localizes to SGs upon heat treatment, disappearing upon stress removal.

### The intrinsically disordered regions of MC1 are aggregation prone and confer insolubility in vitro

A combination of two predictive software was used (D²P², https://d2p2.pro and DISOPRED3, http://bioinf.cs.ucl.uk/psipred; Oates et al., 2013; Jones and Cozzetto, 2015) to pinpoint potential intrinsically disordered regions (IDRs) within MC1 amino acid sequence (SEQ ID NO: 1). Based on these predictions, MC1 encompasses 2 major IDRs (Fig. 2A), one at the N-terminal prodomain and another at a region of the predicted C-terminal p10 catalytic domain known as the 360 loop. Since aggregation propensity is also considered an intrinsic determinant of phase separation, AGGRESCAN3D (A3D) was used to predict the structural aggregation propensity of MC1 on top of its alphafold predicted structure (http://biocomp.chem.uw.edu.pl/A3D/; Zambrano et al., 2015). Interestingly, MC1 displays strong aggregation propensity at the predicted IDRs (Fig. 2B). In particular, the 360 loop shows the longest stretch of amino acids with high aggregation propensity scores.

The 360 loop of MC1 is a highly hydrophobic sequence only present in plant Type I metacaspases. In fungi, protozoa and red algae, Type I metacaspases do not contain the 360 loop and interestingly, these proteins are soluble when full-length is produced recombinantly *in vitro.* In contrast, previous efforts to produce recombinant plant Type I metacaspases proved unsuccessful due to the fact that their full-length versions are highly insoluble. Removal of the 360 loop (amino acids 318-346 of SEQ ID NO: 1) and the prodomain (amino acids 2-77 of SEQ ID NO: 1) was necessary to express soluble MC1 (ΔNMC1Δ360 or rMC1, SEQ ID NO: 2) in *Escherichia coli* (Fig. 3A,B). Removal of the 360 loop alone to generate MC1Δ360 (SEQ ID NO: 5) was not sufficient to solubilize MC1 (Fig. 3B). The soluble MC1 variant devoid of the prodomain and the 360 loop (SEQ ID NO: 2) carrying an N-terminal hexahistidine tag (6xHis) was purified to homogeneity by nickel-affinity chromatography and further isolated by size-exclusion chromatography removing minor impurities (Figure 3C). Importantly, rMC1 was catalytically active as shown by its ability to cleave Arabidopsis SERPIN1, an inhibitor and previously reported *in planta* substrate of MC1. rMC1 behaved as a canonical Type I metacaspase, showing dependency on calcium ions at low millimolar concentrations (1-10 mM) and a neutral pH (pH 7) for maximum cleavage of the fluorogenic substrate Z-FR-AMC. In agreement with the observed trypsin-like activity of metacaspases, rMC1 cleaved the trypsin substrate β-casein (rβ-casein). rMC1 carrying a point mutation in the catalytic cysteine (C220) to alanine (rMC1CA, SEQ ID NO: 5) was also produced and purified. Importantly, rMC1CA was unable to cleave SERPIN1 or rβ-casein. Together, these data show that MC1 contains two distinct IDRs that are aggregation prone and confer high insolubility for protein overexpression and isolation *in vitro.* When removed, proteolytically active and soluble rMC1 could be easily expressed and isolated.

### MC1 can specifically degrade aggregated proteins

First, it was investigated whether Arabidopsis plants lacking MC1 show defects in protein aggregate clearance and survival after proteotoxic stress. To monitor changes in protein aggregation, filter trap analysis was used, a robust method to detect and quantify protein aggregates, in 5-day-old WT and mc1 seedlings after heat stress. Seedlings were subjected to 90' of a moderate heat shock at 37 °C, followed by 90' of recovery at 22 °C and a severe heat shock at 45 °C for 90'. Samples were collected after 1 day of recovery at 22 °C, using non-stressed seedlings as control. Accumulation of aggregated forms of actin, Hsp90 and proteins containing polyQ stretches was analyzed, all of them shown to aggregate in Arabidopsis after heat stress (Llamas *et al.,* 2022). Under basal conditions, protein aggregates (actin, Hsp90-tagged or poly-Q-containing proteins) are barely detectable both in wild-type (WT) and mc1 mutants, as they can be efficiently cleared by PQC mechanisms (Fig. 4A). Stresses such as heat shock result in a sudden overaccumulation of misfolded proteins that often surpasses the PQC capacity of the cell and results in protein aggregation detectable by filter trap (Fig. 4A). Plants lacking MC1 accumulated higher quantities of aggregated proteins than WT after proteotoxic stress, indicating a reduced capacity to manage protein aggregation and proteotoxic stress in the mutant.

Second, it was assessed the capacity of MC1 to disassemble pathological protein aggregates in cell-free conditions. Equimolar concentrations of rMC1 (SEQ ID NO: 2) with aggregates of human transthyretin (TTR) were co-incubated. TTR is a homotetrameric thyroxine transport protein in which tetramer dissociation events lead to aggregation (Quintas *et al.,* 2001). Extracellular insoluble deposits of TTR in several human organs give rise to distinct progressive and fatal clinical syndromes known as transthyretin amyloidosis (Rapezzi et *al.,* 2010). Using turbidity measurements to monitor protein aggregation, it was observed that rMC1 treatment caused a 90 % reduction of TTR aggregates (Fig. 4B). This protein aggregate clearance activity was dependent on MC1 catalytic activity, as evidenced by the absence of disaggregation in the catalytically dead mutant rMC1CA (SEQ ID NO: 6). Indeed, a threefold higher turbidity signal was observed in the rMC1CA-treated samples suggesting that inactive MC1 becomes aggregated when TTR insoluble assemblies are present in the reaction. Visual inspection of TTR samples by transmission electron microscopy (TEM) (Fig 4C) confirmed the disaggregation activity of MC1. The need of catalytic activity for disaggregation is consistent with the observation that aggregated TTR becomes significantly degraded in presence of rMC1 (lane 3), as demonstrated by SDS-PAGE (Fig 4 D). Noteworthy, rMC1 acts as a specific disaggregase, clearing protein aggregates but not the functional form of proteins, since it is unable to degrade soluble TTR in its native tetrameric state (nTTR) (Fig 4D lane 3 and 4 respectively). The obtained data indicate that rMC1 targets and disassembles specifically the aggregated and pathogenic form of TTR. Notably, this disaggregase activity towards TTR aggregates was not observed in samples incubated with MC4 (Fig 4B, D). It was confirmed the activity of the MC4 protease by its rapid autoprocessing in the presence of calcium (Fig 4D lane 10), as previously described.

Finally, it was tested the capacity of MC1 to degrade protein aggregates in cells cultured *in vitro.* To this end it was used two well-established model systems: i) human embryonic kidney (HEK) cells expressing a polyQ-expanded Huntingtin form (Q74) that causes aggregation and proteotoxicity used as a proxy for the neurodegenerative Huntington's disease (Jimenez-Sanchez et al., 2015) and ii) yeast expressing a constitutively misfolded carboxypeptidase (ΔssCPY∗) that forms insoluble protein aggregates upon stress (Park et al., 2007). Co-expression of full-length MC1 (SEQ ID NO: 1) with Q74 fused to mCherry in HEK cells resulted in a reduction of protein aggregates in comparison to expression of Q74 fused to mCherry alone, as demonstrated by filter trap analysis using anti-mCherry antibody (Fig. 4E). In yeast, ΔssCPY∗ was expressed fused to the prototrophic marker Leu2 and a C-terminal myc tag (ΔssCL∗) in wild type (WT), a mutant lacking the single metacaspase *MCA1* in yeast (*ymca1Δ*) and *ymca1Δ* complemented with a WT copy of the Arabidopsis MC1. All strains grew normally on control media, while on selective media lacking leucine WT yeast had reduced growth capacity due to degradation of misfolded ΔssCL∗ by PQC systems (Fig. 5A, right panel and 5B). In contrast, *ymca1Δ* was not able to degrade ΔssCL∗ and therefore could grow normally on a leucine-selective media. This phenotype could be fully complemented by AtMC1 (SEQ ID NO: 1), that due to its ability to degrade ΔssCL∗ restored yeast WT growth levels (Fig. 5A, right panel and 5B). Altogether, these data demonstrate that MC1 can degrade protein aggregates, particularly pathogenic protein aggregates, both in cell-free conditions and inside cells.

### Citation List

Shorter J., "Designer protein disaggregases to counter neurodegenerative disease", 2017, Curr Opin Genet Dev., vol. 44, pp. 1-8
Oates, M.E. et al. (2013) `D2P2: database of disordered protein predictions', Nucleic acids research, 41(Database issue).
Chu, X. et al. (2022) 'Prediction of liquid-liquid phase separating proteins using machine learning', BMC Bioinformatics, 23(1), pp. 1-13.
Zambrano, R. et al. (2015) 'AGGRESCAN3D (A3D): server for prediction of aggregation properties of protein structures', Nucleic Acids Research, 43(W1), pp. W306-W313.
Jumper, J. et al. (2021) 'Highly accurate protein structure prediction with AlphaFold', Nature 2021 596:7873, 596(7873), pp. 583-589.
Llamas, E. et al. (2022) `Chloroplast protein import determines plant proteostasis and retrograde signaling', bioRxiv, p. 2022.03.19.484971.
Quintas, A. et al. (2001) 'Tetramer dissociation and monomer partial unfolding precedes protofibril formation in amyloidogenic transthyretin variants', The Journal of biological chemistry, 276(29), pp. 27207-27213.
Rapezzi, C. et al. (2010) `Transthyretin-related amyloidoses and the heart: a clinical overview', Nature reviews. Cardiology, 7(7), pp. 398-408.
Jimenez-Sanchez, M. et al. (2015) 'siRNA screen identifies QPCT as a druggable target for Huntington's disease.', Nature Chemical Biology, 11(5), pp. 347-354.
Park, S.H. et al. (2007) 'The cytoplasmic Hsp70 chaperone machinery subjects misfolded and endoplasmic reticulum import-incompetent proteins to degradation via the ubiquitin-proteasome system', Molecular Biology of the Cell, 18(1), pp. 153-165.
Gardner, J.M. and Jaspersen, S.L. (2014) 'Manipulating the yeast genome: deletion, mutation, and tagging by PCR', Methods in molecular biology (Clifton, N.J.), 1205, pp. 45-78.
Balaji, V. et al. (2022) 'A dimer-monomer switch controls CHIP-dependent substrate ubiquitylation and processing', Molecular cell, 82(17), pp. 3239-3254.e11.
Lázaro-Silva, D.N. et al. (2015) 'The Use of DNA Extraction for Molecular Biology and Biotechnology Training: A Practical and Alternative Approach', Creative Education, 6(8), pp. 762-772.
Eisele, F. and Wolf, D.H. (2008) 'Degradation of misfolded protein in the cytoplasm is mediated by the ubiquitin ligase Ubr1', FEBS letters, 582(30), pp. 4143-4146.
de Marco, A. et al. (2007) 'Chaperone-based procedure to increase yields of soluble recombinant proteins produced in E. coli', BMC Biotechnology, 7, p. 32.
Vercammen, D. et al. (2004) 'Type II metacaspases Atmc4 and Atmc9 of Arabidopsis thaliana cleave substrates after arginine and lysine', The Journal of biological chemistry, 279(44), pp. 45329-45336.
Pinheiro, F. et al. (2021) `Tolcapone, a potent aggregation inhibitor for the treatment of familial leptomeningeal amyloidosis', The FEBS Journal, 288(1), pp. 310-324.
Coll, N.S. et al. (2010) 'Arabidopsis type I metacaspases control cell death', Science, 330(6009), pp. 1393-1397.
Copolovici D. M. et al., "Cell-Penetrating Peptides: Design, Synthesis, and Applications", 2014, ACS Nano, 2014, 8 (3), pp 1972-1994
Altschul et al., "Basic local alignment search tool", 1990, J. Mol. Biol, v. 215, pages 403-410
EMBOSS: The European Molecular Biology Open Software Suite, Rice et al., 2000, Trends Genet. 16: 276-277

## Claims

1. Polypeptide having disaggregase activity for use in medicine, wherein the polypeptide comprises the sequence of SEQ ID NO: 1 or SEQ ID NO: 2, or a variant thereof that retains the disaggregase activity.

2. Polypeptide having disaggregase activity for use in the prevention or treatment of a disease or disorder associated with protein aggregates in a subject, wherein the polypeptide comprises the sequence of SEQ ID NO: 1 or SEQ ID NO: 2, or a variant thereof that retains the disaggregase activity.

3. The polypeptide for use according to claim 2, wherein the protein aggregates comprise at least one protein selected from the group consisting of prion protein, polyglutamine protein, amyloid precursor protein, alpha-synuclein, superoxide dismutase, transthyretin, tau, immunoglobulin, amyloid-A, low density lipoprotein receptor, crystallin, beta 2-microglobulin, cystatin C, apolipoprotein A1, TDP-43, FUS, islet amyloid polypeptide, ANF, gelsolin, insulin, lysozyme, p53, and fibrinogen.

4. The polypeptide for use according to claim 2 or 3, wherein the disease or disorder mediated by protein aggregates is selected from the group consisting of Alzheimer's disease, transthyretin amyloidosis, cerebral β-amyloid angiopathy, retinal ganglion cell degeneration, bovine spongiform encephalopathy, kuru, Creutzfeldt-Jakob disease, variant Creutzfeldt-Jakob disease, Gerstmann-Straussler-Scheinker syndrome, fatal familial insomnia, frontotemporal dementia, progressive supranuclear palsy, corticobasal degeration, frontotemporal lobar degeneration, frontemporal lobar degeneration, amyotrophic lateral sclerosis, Huntington's disease, familial British dementia, Familial Danish dementia, hereditary cerebral hemorrhage with amyloidosis, CADASIL, Alexander disease, Seipinopathies, familial amyloidotic neuropothy, senile systemic amyloidosis, serpinopathies, AL amyloidosis, AA amyloidosis, type II diabetes, aortic medial amyloidosis, ApoAl amyloidosis, Apoll amyloidosis, ApoAIV amyloidosis, familial amyloidosis of the Finish type, lysozyme amyloidosis, fibrinogen amyloidosis, dialysis amyloidosis, inclusion body myositis/myopathy, cataracts, cancer associated with p53 aggregates, medullary thyroid carcinoma, cardiac atrial amyloidosis, pituitary prolactinoma, hereditary lattice corneal dystrophy, cutaneous lichen amyloidosis, corneal lactoferrin amyloidosis, corneal lactoferrin amyloidosis, pulmonary alveolar proteinosis, odontogenic tumor amyloidosis, seminal vesical amyloid, cystic fibrosis, sickle cell disease, critical illness myopathy, von Hippel-Lindau disease, spinocerebellar ataxia type 1, spinocerebellar ataxia type 2, spinocerebellar ataxia type 3 (Machado-Joseph disease), spinocerebellar ataxia type 6, spinocerebellar ataxia type 7, spinocerebellar ataxia type 17, Angelman syndrome, giant axon neuropathy, and inclusion body myopathy with Paget disease of bone and frontotemporal dementia (IBMPFD).

5. The polypeptide for use according to any one of claims 2-4, wherein the subject is a mammal; particularly a human.

6. The polypeptide for use according to any one of claims 1-5, wherein the polypeptide comprises the sequence of SEQ ID NO: 1 or SEQ ID NO: 2, or a variant thereof that retains the disaggregase activity and that is at least 70 %, at least 71 %, at least 72%, at least 73%, at least 74%, at least 75%, at least 76%, at least 77%, at least 78%, at least 79%, at least 80 %, at least 81 %, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identical to SEQ ID NO: 1 or SEQ ID NO. 2.

7. The polypeptide for use according to any one of claims 1-6, wherein the polypeptide consists of SEQ ID NO: 1 or SEQ ID NO: 2.

8. Expression vector for use in medicine, particularly in the prevention or treatment of a disease or disorder associated with protein aggregates in a subject, wherein the expression vector comprises a nucleic acid that encodes a polypeptide as defined in any of claims 1-7 operatively linked to an expression promoter.

9. The polypeptide for use according to any one of claims 1-7 or the expression vector for use according to claim 8, which is administered in the form of a pharmaceutical composition together with at least one pharmaceutically acceptable excipient, diluent or carrier.

10. Pharmaceutical composition comprising, together with at least one pharmaceutically acceptable excipient, diluent, or carrier:
- a polypeptide having disaggregase activity, wherein the polypeptide comprises the sequence of SEQ ID NO: 1 or SEQ ID NO: 2, or a variant thereof that retains the disaggregase activity;
- a nucleic acid that encodes the polypeptide having disaggregase activity;
- a gene construct comprising the nucleic acid; or
- an expression vector comprising the gene construct.

11. A method for eliminating or preventing the formation of a protein aggregate in a protein-containing composition, the method comprising contacting the protein-containing composition with a polypeptide having disaggregase activity, wherein the polypeptide comprises the sequence of SEQ ID NO: 1 or SEQ ID NO:2, or a variant thereof that retains the disaggregase activity.

12. Use of a polypeptide having disaggregase activity for eliminating or preventing the formation of a protein aggregate in a protein-containing composition, wherein the polypeptide comprises the sequence of SEQ ID NO: 1 or SEQ ID NO: 2, or a variant thereof that retains the disaggregase activity.

13. A method for imaging a protein aggregate in a subject, the method comprising the steps of (i) administering into the subject a detectable quantity of a detectably labelled polypeptide having disaggregase activity, wherein the polypeptide comprises the sequence of SEQ ID NO: 1 or SEQ ID NO: 2, or a variant thereof that retains the disaggregase activity; and (ii) detecting the polypeptide associated with the protein aggregate.

14. Use of a polypeptide having disaggregase activity for imaging a protein aggregate, wherein the polypeptide is a detectably labelled polypeptide comprising the sequence of SEQ ID NO: 1 or SEQ ID NO: 2, or a variant thereof that retains the disaggregase activity.

15. A polypeptide having disaggregase activity for use in the *in vivo* diagnosis or prognosis of a disease or disorder associated with protein aggregates in a subject, wherein the polypeptide is a detectably labelled polypeptide comprising the sequence of SEQ ID NO: 1 or SEQ ID NO:2, or a variant thereof that retains the disaggregase activity.
